# DEMANDE DE BREVET EUROPEEN

(11) **EP 3 902 296 A1**
(43) Date de publication de la demande: **27.10.2021**
(21) Numéro de dépôt: 20020202.6
(22) Date de dépôt: 26.04.2020
(51) Int. Cl.: H04W 4/80, H04B 1/3827, H04W 4/90, F16P 3/12, F16P 3/14, B23Q 5/58, A61F 9/02

(54) **ÉQUIPEMENT DE PROTECTION POUR OPÉRATEURS DE MACHINES À RISQUE**

(71) Demandeur: Santos de Oliveira, Eduardo, 1004 Lausanne (CH)
(72) Inventeur: Santos de Oliveira, Eduardo, 1004 Lausanne (CH)

(57) **Abrégé**

Il est décrit un équipement de protection pour opérateur d'une machine à risque (50), comprenant au moins un accessoire de protection (10) destiné à être porté par l'opérateur lors du maniement de machine à risque (50) par l'opérateur, ledit au moins un accessoire de protection (10) étant équipé d'un dispositif de détection (100) permettent de déterminer si l'accessoire de protection (10) est porté par l'opérateur. L'équipement de protection comprend par ailleurs un dispositif de contrôle (500) destiné à être associé fonctionnellement à la machine à risque (50) devant être maniée par l'opérateur afin d'autoriser ou non un fonctionnement de la machine à risque (50). Le dispositif de détection (100) équipant l'accessoire de protection (10) et le dispositif de contrôle (500) associé à la machine à risque (50) comportent chacun des moyens de communication sans fil (130, 530) configurés pour établir une connexion sans fil entre le dispositif de détection (100) et le dispositif de contrôle (500) afin d'autoriser le fonctionnement de la machine à risque (50) et un maniement de cette machine à risque (50) par l'opérateur dès lors que le dispositif de détection (100) détermine que l'accessoire de protection (10) est porté par l'opérateur.

## Description

### DOMAINE TECHNIQUE

La présente invention se rapporte de manière générale à un équipement électronique de protection pour opérateurs de machines à risque, notamment pour ouvriers du bâtiment, ouvriers de chantier, ouvriers agricoles, artisans-menuisiers, bûcherons-forestiers, jardiniers-paysagistes, ou tous autres opérateurs de machines à risque pour lesquels il convient d'assurer une protection concernant d'éventuels risques de blessure lors du maniement de la machine à risque. La présente invention concerne également une machine à risque configurée pour coopérer avec un tel équipement de protection.

### ARRIÈRE-PLAN TECHNOLOGIQUE

Il est d'usage que tout opérateur d'une machine à risque, qu'il s'agisse d'outillage portatif ou non, soit dûment protéger contre d'éventuels risques de blessures par de l'équipement de protection adéquat. Cet équipement de protection inclut typiquement un ou plusieurs accessoires de protection. Il peut en particulier s'agir d'une paire de lunettes de protection destinée à protéger l'opérateur contre des projections de fragments ou débris produits lors du maniement de la machine à risque, un casque de protection destiné à protéger la tête de l'opérateur contre une chute éventuelle d'objets, un casque anti-bruit destiné à protéger l'ouïe de l'opérateur contre un niveau de bruit trop important dépassant par exemple 90 dB (A), une paire de gants de protection destinée à protéger les mains de l'opérateur, une paire de chaussures de protection destinée à protéger les pieds de l'opérateur, ou tout autre accessoire de protection similaire permettant d'éviter ou de réduire les risques de blessure.

L'on constate malheureusement trop souvent que ces accessoires de protection ne sont pas ou trop peu utilisés en pratique, et ce malgré les recommandations et/ou directives de sécurité normalement en vigueur, ce qui expose les opérateurs à des risques importants.

Une solution permettant d'atténuer, voire éliminer ce problème est donc souhaitable.

### EXPOSÉ DE L'INVENTION

Un but général de la présente invention est donc de proposer une solution permettant d'assurer une meilleure protection à tout opérateur des machines à risque.

Plus particulièrement, un but de la présente invention est de proposer un équipement de protection amélioré qui assure que le ou les accessoires, de protection individuel nécessaires soient correctement portés par tout opérateur de machine à risque.

La présente invention répond à ces buts en proposant un équipement de protection pour opérateur d'une machine à risque dont les caractéristiques sont énumérées à la revendication 1, à savoir un tel équipement de protection comprenant au moins un accessoire de protection destiné à être porté par l'opérateur lors du maniement de machine à risque par l'opérateur. Selon l'invention, ledit au moins un accessoire de protection est équipé d'un dispositif de détection permettent de déterminer si l'accessoire de protection est porté par l'opérateur, et l'équipement de protection comprend par ailleurs un dispositif de contrôle destiné à être associé fonctionnellement à la machine à risque devant être maniée par l'opérateur afin d'autoriser ou non un fonctionnement de la machine à risque. Le dispositif de détection équipant l'accessoire de protection et le dispositif de contrôle associé à la machine à risque comportent chacun des moyens de communication sans fil configurés pour établir une connexion sans fil entre le dispositif de détection et le dispositif de contrôle afin d'autoriser le fonctionnement de la machine à risque et un maniement de cette machine à risque par l'opérateur dès lors que le dispositif de détection détermine que l'accessoire de protection est porté par l'opérateur.

Des modes de réalisations avantageux et/ou préférés font l'objet des revendications dépendantes 2 à 14.

Il est également revendiqué une machine à risque destinée au maniement par un opérateur dont les caractéristiques sont énumérées à la revendication 15, à savoir une telle machine à risque comportant une interface configurée pour recevoir l'unité de contrôle enfichable selon la revendication 13 ou en ce qu'elle incorpore une unité de contrôle intégrée selon la revendication 14.

D'autres aspects de l'invention sont exposés dans la suite de la présente description.

### DESCRIPTION SOMMAIRE DES DESSINS

Les caractéristiques et avantages de la présente invention apparaîtront plus clairement à la lecture de la description détaillée qui suit de modes de réalisation de l'invention, lesquels sont présentés uniquement à titre d'exemples non limitatifs et sont illustrés par les dessins annexés où :
- la Figure 1 est une vue en perspective schématique d'un accessoire de protection, en l'occurrence une paire de lunettes de protection, équipé d'un dispositif de détection selon un mode de réalisation de l'invention ;
- la Figure 2 est une vue en perspective schématique du dispositif de détection équipant la paire de lunettes de protection de la Figure 1 montrant notamment un exemple d'agencement de capteurs ;
- la Figure 3 est un diagramme fonctionnel schématique du dispositif de détection de la Figure 2 ;
- la Figure 4A est une vue en perspective schématique, partielle, d'un autre accessoire de protection, en l'occurrence un casque anti-bruit, équipé d'un dispositif de détection selon un autre mode de réalisation de l'invention ;
- la Figures 4B est une vue en perspective schématique, partielle, du casque anti-bruit de la Figure 4A montrant un exemple d'agencement de capteurs ;
- la Figure 5 est une vue en perspective schématique d'une machine à risque, en l'occurrence un marteau perforateur, équipé d'un dispositif de contrôle selon un mode de réalisation de l'invention ; et
- la Figure 6 est un diagramme fonctionnel schématique du dispositif de contrôle de la Figure 5.

### MODES DE RÉALISATION DE L'INVENTION

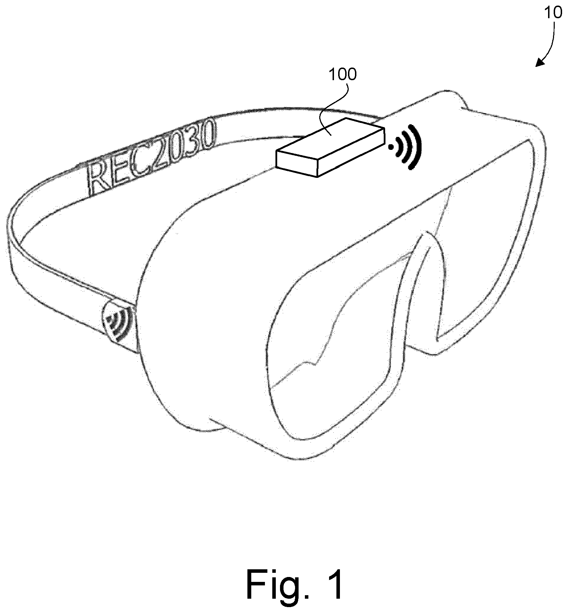

La présente invention sera décrite en référence à divers modes de réalisation préférés tels qu'illustrés notamment par les Figures 1 à 6.

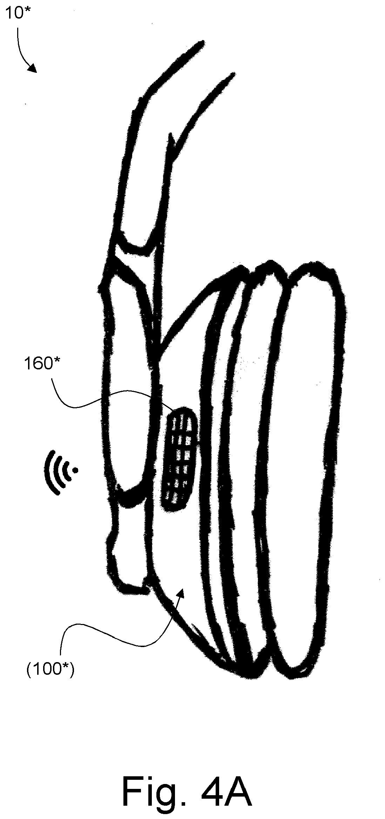

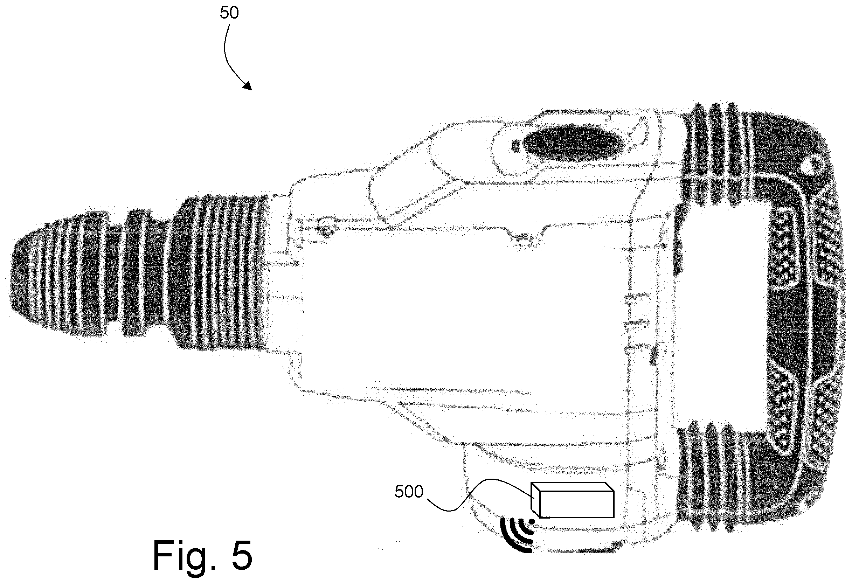

Par « machine à risque », l'on entend de manière générale qu'il est question de toute machine susceptible d'engendrer un risque de blessure à l'opérateur maniant cette machine et qui nécessite typiquement l'adoption par l'opérateur d'un équipement de protection comprenant un ou plusieurs accessoires de protection adéquats. L'invention sera décrite ci-après en rapport à l'utilisation d'un outillage portatif tel un marteau perforateur (voir Figure 5), lequel nécessite typiquement l'utilisation d'une paire de lunettes de protection (voir Figure 1) ainsi que d'un casque anti-bruit (voir Figures 4A-B). Il est toutefois évident que l'évocation d'un marteau perforateur comme exemple de machine à risque n'est nullement limitative et qu'il pourrait s'agir de tout autre outillage portatif tel qu'utilisé notamment dans l'industrie du bâtiment, par exemple un marteau piqueur, une disqueuse, une meuleuse, une perceuse, une visseuse, une cloueuse, une agrafeuse, une scie circulaire, une ponceuse, un rabot, une fraiseuse, une défonceuse, ou tout autre outillage portatif à alimentation pneumatique a combustion ou électrique.

Bien que l'invention soit en particulier utile en rapport au maniement d'un outillage portatif comme évoqué plus haut, l'invention est également applicable par analogie au maniement de toute autre machine à risque. Il pourrait par exemple s'agir d'un engin de chantier, tels une chargeuse, une pelleteuse, une excavatrice, une tractopelle, un bulldozer, un rouleau compresseur, une grue ou autre engin de chantier à risque, ou encore d'un outillage de jardinage, tels une tondeuse à gazon, une débroussailleuse, un sacrificateur, une broyeuse à végétaux, un taille-haie, une tronçonneuse, un motoculteur ou tout autre outillage de jardinage à risque.

La Figure 1 est une vue en perspective schématique d'une paire de lunettes de protection, désignée globalement par la référence 10. Cette paire de lunettes 10 est utilisée comme accessoire de protection destiné à être porté par l'opérateur lors du maniement du marteau perforateur illustré schématiquement à la Figure 5, lequel est désigné globalement par la référence 50.

La paire de lunettes de protection 10 est équipée d'un dispositif de détection 100 permettant de déterminer si cet accessoire de protection est porté par l'utilisateur. Dans le cas d'espèce, le dispositif de détection 100 est placé sur une partie supérieure de la paire de lunettes de protection 10 de manière à pouvoir coopérer avec le front de l'opérateur, comme cela sera détaillé ci-après.

D'une manière générale, le dispositif de détection 100 est configuré pour établir une connexion sans fil avec un dispositif de contrôle qui est associé fonctionnellement à la machine à risque devant être maniée par l'opérateur, à savoir le marteau perforateur 50 de la Figure 5 dans l'exemple considéré. Le dispositif de contrôle est illustré schématiquement dans la Figure 5 et désigné par la référence 500. Ce dispositif de contrôle 500 est associé fonctionnellement au marteau perforateur 50 afin d'autoriser ou non un fonctionnement de cette machine à risque.

Selon l'invention, le dispositif de détection 100 équipant l'accessoire de protection 10 et le dispositif de contrôle 500 associé à la machine à risque 50 comportent chacun des moyens de communication sans fil configurés pour établir une connexion sans fil entre le dispositif de détection 100 et le dispositif de contrôle 500 afin d'autoriser le fonctionnement de la machine à risque 50 et un maniement de cette machine à risque 50 par l'opérateur dès lors que le dispositif de détection 100 détermine que l'accessoire de protection est porté par l'opérateur.

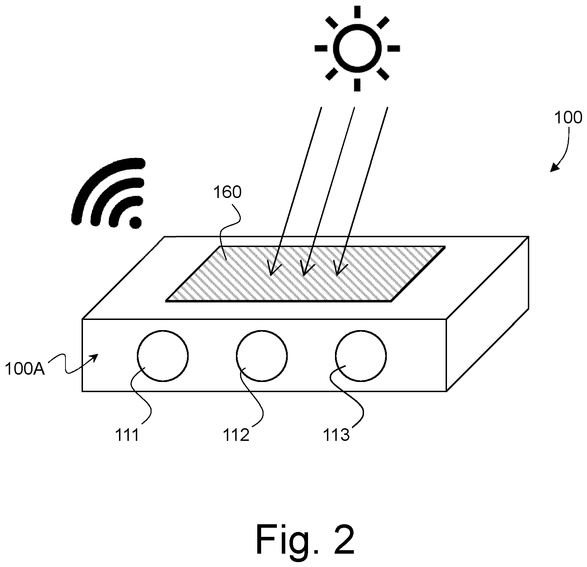

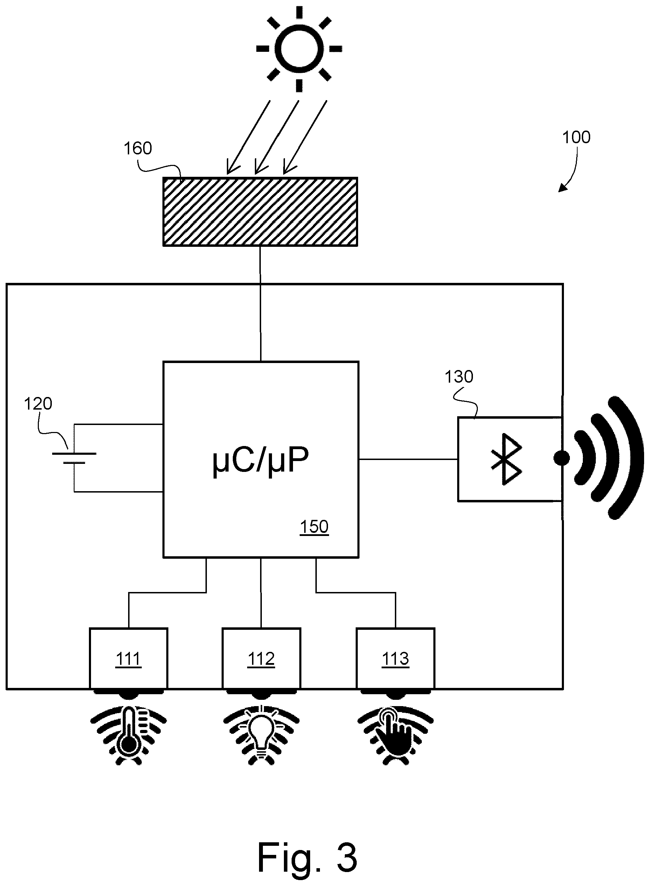

Faisant référence aux Figures 2 et 3, l'on peut constater que le dispositif de détection 100 comporte au moins un capteur, en l'occurrence trois capteurs 111, 112, 113, configurés pour déterminer si la paire de lunettes de protection 10 est portée par l'opérateur. Dans le cas d'espèce, les capteurs 111, 112, 113 sont agencés sur une face 100A du dispositif de détection 100 de manière à venir au contact ou à proximité du front de l'opérateur, une fois la paire de lunettes de protection 10 portée par l'opérateur. Dans l'exemple de réalisation illustré, le premier capteur 111 est préférablement un capteur thermique, le deuxième capteur 112 un capteur de luminosité et le troisième capteur 113 un capteur de proximité.

Plus spécifiquement, le capteur thermique 111 est placé sur l'accessoire de protection 10 de telle sorte à mesurer une température de l'opérateur lorsque l'accessoire de protection est porté par l'opérateur, fournissant ainsi une première mesure permettant de vérifier si la paire de lunettes de protection 10 est portée par l'opérateur.

Le capteur de luminosité 112 est quant à lui placé sur l'accessoire de protection 10 de telle sorte à obscurcir le champ de mesure du capteur lorsque l'accessoire de protection est porté par l'opérateur, fournissant ainsi une seconde mesure permettant de vérifier si la paire de lunettes de protection 10 est portée par l'opérateur.

Enfin, le capteur de proximité 113 est placé sur l'accessoire de protection 10 de telle sorte à détecter un contact ou proximité entre l'accessoire de protection 10 et l'opérateur lorsque l'accessoire de protection 10 est porté par l'opérateur, fournissant ainsi une troisième mesure permettant de vérifier si la paire de lunettes de protection 10 est portée par l'opérateur.

Les Figures 2 et 3 montrent également la présence optionnelle d'une cellule photovoltaïque 160 permettant d'assurer une alimentation du dispositif de détection 100, voire une recharge d'une batterie 120 (voir Figure 3) alimentant le dispositif de détection 100.

Faisant plus spécifiquement référence au diagramme fonctionnel schématique de la Figure 3, l'on peut voir que le dispositif de détection 100 comporte un microcontrôleur (ou microprocesseur) 150 programmé pour procéder au traitement des signaux des trois capteurs 111, 112, 113 et déterminer si ces signaux sont indicatifs du fait que l'accessoire de protection 10 est porté par l'opérateur. Ce microcontrôleur (ou microprocesseur) 150 est en communication avec un émetteur-récepteur 130 configuré pour permettre l'établissement d'une communication sans fil, en particulier une communication courte-distance (préférablement sécurisée), entre le dispositif de détection 100 et le dispositif de contrôle 500 associé à la machine à risque 50.

Les Figures 1 et 2 montrent le dispositif de détection 100 comme un dispositif dédié qui est apposé sur la partie supérieure de la paire de lunettes de protection 10. L'on comprendra que les fonctions du dispositif de détection 100 pourraient être réalisées de toute autre manière adéquate, par exemple en intégrant ces fonctions directement dans la paire de lunettes de protection 10. Les capteurs 111, 112, 113 pourraient ainsi être disposés en tout autre endroit adéquat, par exemple au niveau du support nasal, ou de la bande de retenue entourant la tête de l'opérateur. La disposition des capteurs dépendra fondamentalement de la configuration de l'accessoire de protection considéré et des possibilités de positionnement qui sont offertes à cet égard.

Les Figures 4A-B sont des vues en perspective schématiques, partielles, d'un casque anti-bruit, désigné globalement par la référence 10*. Ce casque anti-bruit 10* est utilisée comme accessoire de protection complémentaire destiné à être porté par l'opérateur lors du maniement du marteau perforateur 50 illustré schématiquement à la Figure 5.

À l'image de la paire de lunettes de protection 10, le casque anti-bruit 10* est équipé d'un dispositif de détection 100* permettant de déterminer si cet accessoire de protection est porté par l'utilisateur. Dans le cas d'espèce, le dispositif de détection 100* est intégré à l'intérieur de l'une des deux coquilles du casque anti-bruit 10* de manière à pouvoir coopérer avec la partie englobant l'oreille de l'opérateur.

Le dispositif de détection 100* est de même configuré pour établir une connexion sans fil avec le dispositif de contrôle 500 qui est associé fonctionnellement à la machine à risque devant être maniée par l'opérateur, à savoir le marteau perforateur 50 de la Figure 5 dans l'exemple considéré.

La Figure 4A montre schématiquement la présence d'une cellule photovoltaïque 160* placée sur la coquille du casque anti-bruit 10* et permettant d'assurer une alimentation du dispositif de détection 100*, voire une recharge d'une batterie rechargeable alimentant le dispositif de détection 100* (à l'image du dispositif de détection 100).

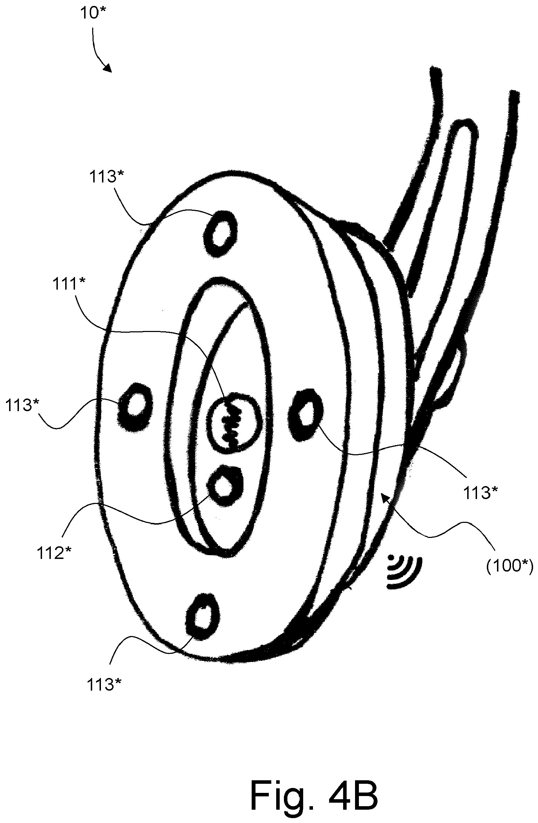

Comme illustré dans la Figure 4B, le dispositif de détection 100* comporte de même une pluralité de capteurs, en l'occurrence un capteur thermique 111* et un capteur de luminosité 112* tous deux logés dans la partie centrale de l'élément de la coquille qui vient normalement se placer sur et autour de l'oreille de l'opérateur, ainsi que quatre capteurs de proximité 113* distribués sur la partie périphérique de cet élément. Les capteurs 111*, 112*, 113* sont également configurés pour déterminer si le casque anti-bruit 10* est porté par l'opérateur. Les capteurs 111*, 112*, 113* fournissent des mesures qui permettent une fois de plus de vérifier si le casque anti-bruit 10* est porté par l'opérateur.

Le dispositif de détection 100* opère selon un fonctionnement analogue au dispositif de détection 100 précédemment décrit, étend entendu que le dispositif de détection 100* intègre de même un microcontrôleur (ou microprocesseur), non représenté, programmé pour procéder au traitement des signaux des capteurs 111*, 112*, 113* et déterminer si ces signaux sont indicatifs du fait que l'accessoire de protection 10* est porté par l'opérateur. Le dispositif de détection 100* intègre également un émetteur-récepteur, non représenté, configuré pour permettre l'établissement d'une communication sans fil, en particulier une communication courte-distance (préférablement sécurisée), entre le dispositif de détection 100* et le dispositif de contrôle 500 associé à la machine à risque 50.

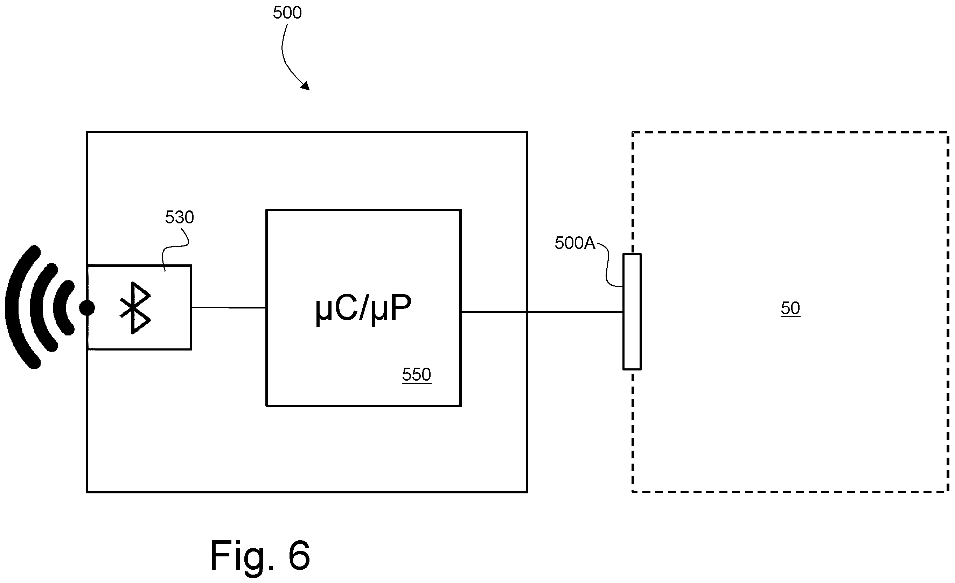

La Figure 6 montre un diagramme fonctionnel schématique du dispositif de contrôle 500 associé fonctionnellement à la machine à risque 50. Dans le cas d'espèce, le dispositif de contrôle 500 est préférablement configuré comme une unité de contrôle enfichable raccordée à la machine à risque 50 par l'intermédiaire d'une interface 500A (par exemple une interface USB) afin de contrôler et autoriser ou non son fonctionnement. À cet égard, le dispositif de contrôle 500 est ici assimilable à une clé matérielle (ou « dongle » en terminologie anglosaxonne) permettant de verrouiller ou déverrouiller le fonctionnement de la machine à risque 50.

En lieu et place d'une unité de contrôle enfichable, le dispositif de contrôle 500 pourrait alternativement être configuré comme une unité de contrôle intégrée à la machine à risque 50 afin de contrôler et autoriser ou non son fonctionnement. Dans ce cas, le dispositif de contrôle 500 serait ici assimilable à une clé matérielle intégrée à la machine à risque 50.

Indépendamment de ce qui précède, le dispositif de contrôle 500 comporte un émetteur-récepteur 530 configuré pour permettre l'établissement d'une communication sans fil, en particulier une communication courte-distance, avec chaque dispositif de détection 100, resp. 100*, équipant le ou les accessoires de protection 10, 10*, lequel émetteur-récepteur 530 est couplé à un microcontrôleur (ou microprocesseur) 550 programmé pour procéder au traitement des signaux reçus de ou devant être transmis à l'émetteur-récepteur 530.

Les émetteurs-récepteurs susmentionnés 130, 530 sont préférablement des émetteurs-récepteurs Bluetooth, à savoir des émetteurs-récepteurs courte-distance utilisant des ondes radio UHF (2.4 GHz) opérant selon la norme Bluetooth (norme IEEE 802.15.1), d'autres normes ou modes de communication sans fil étant toutefois éventuellement envisageables.

À titre préféré, chaque dispositif de détection et le dispositif de contrôle sont préférablement configurés pour procéder au préalable à un jumelage (ou « appairement ») assurant que chaque dispositif de détection équipant l'accessoire de protection devant être porté par l'opérateur est spécifiquement jumelé au dispositif de contrôle associé à la machine à risque devant être maniée par l'opérateur. Ce processus de jumelage préalable peut être réalisé par un processus d'authentification semi-automatisé dit de « challenge-and-response » initié par le dispositif de détection ou par le dispositif de contrôle.

Dans l'exemple considéré, l'on comprendra donc que l'équipement de protection comprend deux accessoires de protection, à savoir la paire de lunettes de protection 10 et le casque anti-bruit 10*, devant être portés conjointement par l'opérateur pour permettre le maniement de la machine à risque, à savoir le marteau perforateur 50. En d'autres termes, chaque accessoire de protection 10, resp. 10*, est équipé d'un dispositif de détection 100, resp. 100*, permettant de déterminer si chaque accessoire de protection est porté par l'opérateur, et la connexion sans fil est établie entre chaque dispositif de détection 100, resp. 100*, et le dispositif de contrôle 500 associé à la machine à risque 50 afin d'autoriser le fonctionnement de la machine à risque 50 et le maniement de cette machine par l'opérateur dès lors que chaque dispositif de détection 100, resp. 100*, détermine que l'accessoire de protection associé 10, resp. 10*, est porté par l'opérateur.

L'on comprendra de manière générale que diverses modifications et/ou améliorations évidentes pour l'homme du métier peuvent être apportées aux modes de réalisation décrits dans la présente description sans sortir du cadre de l'invention défini par les revendications annexées. Par exemple, l'invention n'est pas limitée au maniement d'outillage portatif à risque, tel le marteau perforateur de la Figure 5, mais est également applicable au maniement de toutes autres machines à risque, tel un engin de chantier ou un outillage de jardinage.

### LISTE DES SIGNES DE RÉFÉRENCE UTILISÉS DANS LA PRÉSENTE DESCRIPTION ET DANS LES DESSINS

- 10: paire de lunettes de protection (accessoire de protection)
- 10*: casque anti-bruit (accessoire de protection)
- 50: marteau perforateur (machine à risque)
- 100: dispositif de détection équipant la paire de lunettes de protection 10
- 111: capteur de température
- 112: capteur de luminosité
- 113: capteur de contact
- 120: batterie (rechareable)
- 130: émetteur-récepteur pour établissement d'une communication sans fil à courte distance (par ex. RF, Bluetooth. etc...) avec le dispositif de contrôle 500
- 150: microcontrôleur/ microprocesseur
- 160: cellule photovoltaïque
- 100*: dispositif de détection équipant le casque anti-bruit 10*
- 111*: capteur de température
- 112*: capteur de luminosité
- 113*: capteurs de contact
- 160*: cellule photovoltaïque
- 500: dispositifs de contrôle équipant le marteau perforateur 50
- 500A: interface (par ex. USB) pour raccordement du dispositif de contrôle ou recharge 500 à la machine à risque (par ex. marteau perforateur) 50
- 530: émetteur-récepteur pour établissement d'une communication sans fil à courte distance (par ex. Bluetooth) avec le dispositif de détection 100, 100*
- 550: microcontrôleur / microprocesseur

## Revendications

1. Équipement de protection pour opérateur d'une machine à risque (50), comprenant au moins un accessoire de protection (10 ; 10*) destiné à être porté par l'opérateur lors du maniement de machine à risque (50) par l'opérateur,
**caractérisé en ce que** ledit au moins un accessoire de protection (10 ; 10*) est équipé d'un dispositif de détection (100 ; 100*) permettent de déterminer si l'accessoire de protection (10 ; 10*) est porté par l'opérateur,
**en ce que** l'équipement de protection comprend par ailleurs un dispositif de contrôle (500) destiné à être associé fonctionnellement à la machine à risque (50) devant être maniée par l'opérateur afin d'autoriser ou non un fonctionnement de la machine à risque (50),
et **en ce que** le dispositif de détection (100 ; 100*) équipant l'accessoire de protection (10 ; 10*) et le dispositif de contrôle (500) associé à la machine à risque (50) comportent chacun des moyens de communication sans fil (130, 530) configurés pour établir une connexion sans fil entre le dispositif de détection (100 ; 100*) et le dispositif de contrôle (500) afin d'autoriser le fonctionnement de la machine à risque (50) et un maniement de cette machine à risque (50) par l'opérateur dès lors que le dispositif de détection (100 ; 100*) détermine que l'accessoire de protection (10 ; 10*) est porté par l'opérateur.

2. Équipement de protection selon la revendication 1, **caractérisé en ce que** le dispositif de détection (100 ; 100*) comporte au moins un capteur (111, 112, 113 ; 111*, 112*, 113*) configuré pour déterminer si l'accessoire de protection (10 ; 10*) est porté par l'opérateur, en particulier au contact ou à proximité de la peau de l'opérateur.

3. Équipement de protection selon la revendication 2, **caractérisé en ce que** ledit au moins un capteur (111, 112, 113 ; 111*, 112*, 113*) est un capteur thermique (111 ; 111*), un capteur de luminosité (112 ; 112*) ou un capteur de proximité (113 ; 113*).

4. Équipement de protection selon la revendication 3, **caractérisé en ce que** le dispositif de détection (100 ; 100*) comporte au moins deux capteurs de types différents (111, 112, 113 ; 111*, 112*, 113*), préférablement au moins trois capteurs, sélectionnés parmi les capteurs suivants :
- un capteur thermique (111 ; 111*) placé sur l'accessoire de protection (10 ; 10*) de telle sorte à mesurer une température de l'opérateur lorsque l'accessoire de protection (10 ; 10*) est porté par l'opérateur ;
- un capteur de luminosité (112 ; 112*) placé sur l'accessoire de protection (10 ; 10*) de telle sorte à obscurcir le champ de mesure du capteur de luminosité (112 ; 112*) lorsque l'accessoire de protection (10 ; 10*) est porté par l'opérateur ; et
- un capteur de proximité (113 ; 113*) placé sur l'accessoire de protection (10 ; 10*) de telle sorte à détecter un contact ou proximité entre l'accessoire de protection (10; 10*) et l'opérateur lorsque l'accessoire de protection (10 ; 10*) est porté par l'opérateur.

5. Équipement de protection selon l'une quelconque des revendications précédentes, **caractérisé en ce que** le dispositif de détection (100 ; 100*) est alimenté par une batterie (120), notamment une batterie rechargeable.

6. Équipement de protection selon la revendication 5, **caractérisé en ce que** la batterie (120) est une batterie rechargeable qui peut être rechargée sur le secteur et/ou au moyen d'une cellule photovoltaïque (160 ; 160*) placée sur l'accessoire de protection (10 ; 10*).

7. Équipement de protection selon l'une quelconque des revendications précédentes, **caractérisé en ce que** lesdits moyens de communication sans fil (130 ; 530) sont configurés pour assurer une communication courte-distance, préférablement sécurisée, entre le dispositif de détection (100 ; 100*) équipant l'accessoire de protection (10 ; 10*) et le dispositif de contrôle (500) associé à la machine à risque (50).

8. Équipement de protection selon la revendication 7, **caractérisé en ce que** la communication courte-distance est opérée selon la norme de communication Bluetooth.

9. Équipement de protection selon l'une quelconque des revendications précédentes, **caractérisé en ce que** le dispositif de détection (100 ; 100*) équipant l'accessoire de protection (10 ; 10*) et le dispositif de contrôle (500) associé à la machine à risque (50) sont configurés pour procéder au préalable à un jumelage assurant que le dispositif de détection (100 ; 100*) équipant l'accessoire de protection (10; 10*) porté par l'opérateur est spécifiquement jumelé au dispositif de contrôle (500) associé à la machine à risque (50) devant être maniée par l'opérateur.

10. Équipement de protection selon l'une quelconque des revendications précédentes, **caractérisé en ce que** l'accessoire de protection est une paire de lunettes de protection (10), un casque de protection, un casque anti-bruit (10*), une paire de gants de protection, une paire de chaussures de protection ou un autre accessoire ou vêtement de protection.

11. Équipement de protection selon la revendication 10, dans lequel l'accessoire de protection est une paire de lunettes de protection (10) équipée d'un jeu de capteurs (111, 112, 113) disposés de telle sorte à déterminer si la paire de lunettes de protection (10) est portée par l'opérateur,
ou dans lequel l'accessoire de protection est un casque anti-bruit (10*) équipé d'un jeu de capteurs (111*, 112*, 113*) disposés de telle sorte à déterminer si le casque anti-bruit (10*) est porté par l'opérateur.

12. Équipement de protection selon l'une quelconque des revendications précédentes, **caractérisé en ce qu'**il comporte une pluralité d'accessoires de protection (10, 10*) devant être portés par l'opérateur, chaque accessoire de protection (10, 10*) étant équipé d'un dispositif de détection (100, 100*) permettent de déterminer si chaque accessoire de protection (10, 10*) est porté par l'opérateur,
et **en ce que** la connexion sans fil est établie entre chaque dispositif de détection (100, 100*) et le dispositif de contrôle (500) associé à la machine à risque (50) afin d'autoriser le fonctionnement de la machine à risque (50) et un maniement de cette machine à risque (50) par l'opérateur dès lors que chaque dispositif de détection (100, 100*) détermine que l'accessoire de protection (10, 10*) associé est porté par l'opérateur.

13. Équipement de protection selon l'une quelconque des revendications précédentes, **caractérisé en ce que** le dispositif de contrôle (500) est configuré comme une unité de contrôle enfichable opérant comme clé matérielle susceptible d'être raccordée à une interface (500A) de la machine à risque (50) afin de contrôler et autoriser ou non son fonctionnement.

14. Équipement de protection selon l'une quelconque des revendications 1 à 12, **caractérisé en ce que** le dispositif de contrôle (500) est configuré comme une unité de contrôle intégrée opérant comme clé matérielle intégrée à la machine à risque (50) afin de contrôler et autoriser ou non son fonctionnement.

15. Machine à risque (50) destinée au maniement par un opérateur, **caractérisée en ce qu'**elle comporte une interface (500A) configurée pour recevoir l'unité de contrôle enfichable (500) selon la revendication 13 ou **en ce qu'**elle incorpore une unité de contrôle intégrée (500) selon la revendication 14.
